# EUROPEAN PATENT SPECIFICATION

(11) **EP 1 429 769 B1**
(45) Date of publication and mention of the grant of the patent: **14.01.2009**
(21) Application number: 02799530.7
(22) Date of filing: 10.09.2002
(51) Int. Cl.: A61K 31/465, A61K 9/20, A61K 9/48, A61P 25/34

(54) **NEW FORMULATIONS AND USE THEREOF**
NEUE FORMULIERUNGEN UND IHRE VERWENDUNG
NOUVELLES FORMULATIONS ET UTILISATION DE CES DERNIERES

(30) Priority: 27.09.2001 SE 0103211
(43) Date of publication of application: 23.06.2004
(73) Proprietor: McNeil AB, 254 42 Helsingborg (SE)
(72) Inventor: LINDBERG, Nils-Olof, S-216 15 Malmö (SE)
(74) Representative: Hedenström, John Allan
(86) International application number: PCT/SE2002/001611
(87) International publication number: WO 2003/026655

(56) References cited:
- WO-A1-00/30641
- FR-A1- 2 717 387
- US-A- 5 662 920

## Description

### Field of the Invention

This invention relates to novel pharmaceutical compositions of nicotine and use thereof. More particularly, the present invention relates to compositions comprising nicotine and cocoa powder, methods to prepare said compositions, and to the use of said composition in the manufacture of a medicament for nicotine replacement therapy (NRT), including tobacco substitution and smoking cessation.

### Background and Prior Art

Nicotine replacement therapy as a smoking cessation strategy has been successful in the past. Previous nicotine-containing compositions aiming towards the purpose of reducing nicotine craving for subjects wishing to stop their use of tobacco products include e g US 3,845,217 disclosing chewable compositions, US 4,579,858 disclosing high-viscous nicotine nose-drop compositions, US 5,525,351 disclosing nicotine-containing saliva-soluble gels, US 5,656,255 disclosing low-viscous nicotine-containing compositions suitable for nasal spray administration, US 4,920,989, US 4,953,572 and US 5,167,242 disclosing the use of inhalation aerosol, BP 1,528,391 and BP 2,030,862 disclosing liquid aerosol formulations adapted as mouth-sprays, and devices for transdermal delivery of nicotine.

A well-known side effect of nicotine is related to its concentration dependent local irritation. This adverse effect is particularly noticeable when nicotine formulations are applied topically, including the transmucosal, comprising buccal and nasal, and transdermal administration routes.

UK Patent application GB 2 230 439 A describes nicotine lozenges with a shell or coating containing an oral-acting local analgesic, preferably eugenol. Though not stated explicitly to be the cause of the so included local analgesic, the aforesaid disclosure is said to substantially ameliorate the sensation of burning in the mouth experienced with conventional nicotine lozenges. Similarly, nicotine-compositions formulated in lozenges containing local analgesic have been disclosed in AU 662877 in which the latter agent is said to temporarily interfere with taste receptors which is said to reduce the desire to eat.

The concentration of nicotine in several of the above-mentioned inventions, and product designs thereof, is hence limited by adverse effects caused by or related to its local irritation.

Prior art describes other capsules, tablets, and lozenges for oral delivery of nicotine. For example, WO 88/03803 discloses a chewable capsule filled with a liquid containing 0.1 - 10.0 mg of nicotine, together with additives for improving flavor and dispersion. The capsules are provided in a variety of pH values to allow the patient a choice of nicotine absorption rates, and are especially intended as an aid to quit smoking.

Another nicotine capsule formulation is disclosed by Jarvik et al. (Clinical Pharmacology and Therapeutics 1970; 11: 574) for ingestion as a smoking cessation aid. The subjects, according to the theory that intestinal absorption of nicotine could produce significant blood levels, however, apparently swallowed these capsules whole. The study showed a small but significant decrease in the number of cigarettes smoked by subjects, but no quantitative measurements of nicotine blood levels were obtained.

BE 899037 discloses a tablet containing 0.1 to 5 mg nicotine as a base or watersoluble acid salt as an aid for quitting smoking.

Shaw (in GB 2 142 822 and US 4,806,356) describes a nicotine lozenge prepared from a mixture of inert filler material, a binder, and either pure nicotine or a nicotine-containing substance by cold compression.

US 5,512,306 discloses a nicotine product for oral delivery in the form of an inclusion complex of nicotine and a cyclodextrin compound. It also discusses the use of various excipients and direct compression for manufacture of the product.

WO 97/42941 discloses a slowly erodible nicotine lozenge that allows delivery to the buccal mucosa over an extended period of time.

US 5,662,920 discloses a nicotine lozenge that may contain candy taste flavorants, such as chocolate, orange, vanilla, as well as other flavorants. No amount sufficient for taste-masking is though suggested. Further, cocoa powder is not disclosed.

The literature also describes different designs of tablets for delivering nicotine to the mouth and digestive system.

Wesnes and Warburton (Psychopharmacology 1984; 82:147; *ibid.* 1986; 89:55) discuss the use of nicotine containing dextrose and magnesium hydroxide tablets. The subjects were instructed to keep the tablets in the mouth for some minutes before swallowing, in order to maximize contact with the buccal mucosa.

Several products based on the above mentioned patents are now marketed on an international scale. In addition, several nicotine lozenges are available as over-the-counter products in the UK Resolution lozenges, manufactured by Phoenix Pharmaceuticals and distributed by Ernest Jackson, contain 0.5 mg nicotine, together with the antioxidant vitamins A, C and E. Stoppers lozenges, distributed by Charwell Pharmaceuticals Ltd., contain 0.5 mg nicotine and are available in chocolate, orange and peppermint flavors.

There are, however, subjects who may have cravings for higher doses of nicotine than those acceptable in applications of prior art and subjects that may not experience a decrease in other withdrawal symptoms because of unsatisfactory nicotine absorption. Furthermore, it has to date been difficult to deliver nicotine in a profile mimicking the nicotine blood levels achieved by consistent smoking, to satisfy cravings for nicotine in people who are attempting to quit smoking, and thus, to provide greater protection against relapse than nicotine replacement therapies is possible with hitherto known. Thus, absorption of nicotine in the use of currently marketed products and as disclosed in prior art of nicotine replacement therapies does not satisfactorily resemble the use of tobacco products, in particular smoking. With chewing gum nicotine replacement therapy for smoking cessation blood peak levels of nicotine is reached after 30 minutes with venous blood nicotine levels about 1/3 to 2/3 of the levels attained when smoking (British Medical Journal 1976;1:1043). A smoker will usually reach peak blood levels of nicotine 5 - 10 minutes after starting smoking. It is therefore desirable to provide improved compositions and methods which avoid the disadvantages of these conventional nicotine delivery devices and methods while providing an effective means for delivering nicotine for smoking cessation treatment, for reducing nicotine craving, and for treating other conditions responsive to nicotine therapy.

An attempt to solve the captioned problems is made with a nicotine-containing composition, preferably for buccal uptake, according to WO 00/30641. Herein is disclosed a composition comprising nicotine, at least one apolar component, at least one polar component and at least one surface-active component. Many apolar components are suggested, including lipids such as cocoa butter and cocoa butter alternatives, including cocoa butter equivalents (CBE), cocoa butter substitutes (CBS), cocoa butter replacers (CBR) and cocoa butter improvers (CBI). Anyhow, the composition according to WO 00/30641 has the disadvantage of insufficient taste-masking of nicotine and buffering agents, and the drawback of causing nausea with some users. Cocoa powder is mentioned in one example, where the percentage though is so low that the cocoa powder may serve only as flavorant, not as taste-masking agent.

It has now surprisingly been found that a rapid buccal absorption of nicotine concomitantly with sufficient soothening of the burning sensation of nicotine and sufficient taste-masking of badly tasting ingredients, such as buffering agents, is achieved through the use of nicotine-containing formulations comprising cocoa powder as taste-masking agent, also serving as filler/diluent and smoothening/flavoring agent. No similar formulations have been disclosed hitherto.

A few patent applications disclose cocoa powder as an excipient in different formulations, for example WO 00/51570 disclosing a drug-containing soft capsule comprising cocoa powder, primarily intended for swallowing and drug uptake in the stomach. JP 200095710 discloses compacted tablets comprising cocoa powder and vitamins or iron compounds. WO 00/13523 discloses an encapsulated matrix composition comprising caffeine and a fairly low percentage of cocoa powder. ES 21059710 and WO 95/24890 disclose formulations with certain antibiotics and cocoa powder. JP 93010326 discloses an oily formulation wherein cocoa powder *per se* is the active ingredient.

Chocolate, which is very different from cocoa powder as such, is very rarely used as an ingredient in pharmaceutical products, hitherto only in laxatives. One example is Ex-Lax^{®} being chocolated laxative pieces marketed by Novartis comprising sennosides. In the 1950s was marketed Purex, a laxative wherein phenolphthalein was formulated with chocolate. The Stoppers lozenges mentioned above do not comprise chocolate, or cocoa, but only chocolate flavors. Such chocolate flavors are not useful for the objectives of the present invention.

### Summary of the invention

Compositions for the therapeutic delivery of nicotine are provided. Said compositions comprising nicotine provide rapid transmucosal absorption of nicotine. The compositions are preferably used for therapeutic administration of nicotine.

The meaning of "disintegration" as used in the description and in the claims denotes melting, solubilization, erosion or a combinatorial effect of these physical changes of the invention.

In the absence of explicit statements to the contrary, as used herein expressions like "comprising", "including", "having", "with" and similar terminology shall not be understood to be exclusively restricted to the recited element(s), but shall be understood to allow for the presence of further elements as well, and shall be understood to cover any element(s) in integral, sub-divided or aggregate forms, as well to imply the inclusion of a stated integer or step or group of integers or steps, but not the exclusion of any other integer or step or group of integers or steps.

An object of the invention is to provide new pharmaceutical compositions of nicotine for uptake buccaly or by other mucosa in the oral cavity, comprising a large percentage of cocoa powder.

A second object of the invention is the manufacture of a medicament for nicotine replacement therapy (NRT), including tobacco substitution and smoking cessation.

### Detailed Description of the Invention

It is the primary object of the present invention to provide a tobacco supplement or a tobacco substitute, for use in e g smoking cessation and nicotine replacement therapies which provide the user with a satisfactory dose of nicotine so as to reduce tobacco withdrawal symptoms without causing unacceptable adverse effects. More specifically it is the object of the invention to provide such a nicotine containing tablet, for transmucosal, preferably buccal, delivery, which disintegrates and/or melts at body temperature with or without the aid of salivary fluid or mechanical erosion, or a combination thereof after which the formulation preferably shows adhesiveness towards the tissues in the oral cavity.

In the present invention cocoa powder primarily serves as taste-masking agent, but also serves as diluent, as filler, as agent for providing a smooth texture and as flavorant.

The preferred formulation is a tablet, weighing around 400 mg, having the following preferred composition:
Nicotine (as base or hydrogen tartrate) 1- 6 mg measured as base
Cocoa powder around 50%
Fatty components around 44%
Aspartame around 0.6%
Sodium carbonate around 15 mg
Lecithin around 1%
The percentages are w/w.

Cocoa nib is defined as cocoa beans with the shell removed. Cocoa mass is defined as cocoa nib ground to give a substance being a liquid above 35°C. Cocoa liquor is another name for cocoa mass. Cocoa powder is defined as cocoa nib with some fat removed and ground into a powder. Cocoa butter is defined as fat expelled from the center (kernels or nib) of cocoa beans.

Cocoa powder is prepared from roasted cocoa beans. It is a complex compound, which consists of starch, cocoa butter, amino acids, proteins, xanthines, amines, mono- and polysaccharides, phospholipids, flavonoids, pyrazines, etc.

Preferred fatty components are fats/lipids chosen from tempering fats, including cocoa butter equivalents (CBE) and cocoa butter improvers (CBI), and non-tempering fats, including cocoa butter replacers (CBR) and cocoa butter substitutes (CBS).

It is important to note that there is an essential difference between chocolate and cocoa powder as such. According to Industrial Chocolate Manufacture and Use, S. T. Beckett, ed., 2nd edition, Blackier Academic & Professional, London, 1994, p 382, chocolate is defined as a product obtained from cocoa nib, cocoa mass powder and sucrose with or without added cocoa butter, having a minimum dry cocoa solids content of 35%, at least 14% of dry non-fat cocoa solids and 18% cocoa butter. Chocolate has two major distinguishing characteristics: its flavor and its texture. A primary feature of the texture is that the chocolate must be solid at a temperature of 20 - 25°C and yet melt rapidly in the mouth at 37°C thereby being transferred to a liquid, which appears smooth to the tongue. The processing of chocolate is related to obtaining these two criteria (*ibid*. p 2). Chocolate as such according to the definition above is not suitable in the formulation according to the present invention.

Neither milk chocolate nor light cooking chocolate or dark cooking chocolate may mask the disagreeable taste of most buffering agents. The cocoa content of milk chocolate is comparatively low (a cocoa mass content of 10 - 16%, corresponding to approximately 5 - 8% cocoa powder). The beans'/cocoa mass'content of dark, bitter-sweet chocolate is 55 - 70% (Beckett, pp. 276 - 277), corresponding to approximately 28 - 35% cocoa powder. By making a vehicle with a high proportion of cocoa powder (30 - 50%) and fatty components (40 - 45%), as per the present invention, an effective masking is though obtained. The higher the cocoa powder concentration the better the taste-masking.

### Example 1: Preparation of a preferred embodiment

A tablet, weighing around 400 mg, having the following preferred composition (w/w):

| | |
|---|---|
| Active: | nicotine (as base or salt, preferably hydrogen tartrate) |
| | 1- 6 mg measured as base |
| | The nicotine may also be present in a complex, e g with a |
| | cation exchange resin or with cyclodextrin. |
| Diluent/filler, | |
| taste-masking, | |
| smoothening and | |
| flavoring agent: | cocoa powder around 50% |
| Lipid ingredient: | fatty components around 44% |
| Buffering agent: | sodium carbonate around 15 mg |
| Sweetener: | aspartame around 0,6% |
| Emulsifier/solubilizer: | lecithin around 1% |
| Optional flavoring | |
| agent: | peppermint or vanilla flavor 0,5% |

is prepared in the following way:

A part of the fatty components is melted. The solid components, i e nicotine, if in salt form, cocoa powder, aspartame, sodium carbonate and the optional flavoring agent if solid are added and mixed. A reduction of particle size of the solid components is performed by milling in a roll-refiner. If the solid components have already got the required particle size, e g by milling before the mixing with the fatty components, roll refining is dispensed with. After treatment in the roll-refiner the mixture is mixed with the rest of the melted fatty components or remelted (if solidified) and mixed with the rest of the melted fatty components. A mixing of the melt is performed in a suitable mixer. The liquid components, i e lecithin, nicotine, if in the liquid base form, and the optional flavoring agent if liquid, are added. Tablets or other solid dosage forms are subsequently made using suitable techniques, such as molding, extrusion or congealing, including pastillation, when necessary after suitable preconditioning. Also other suitable manufacturing methods may be used.

### Example 2: Further embodiments

Useful embodiments are obtained by exchanging some of the above-mentioned excipients for equivalently functioning alternative compounds, selected from the following list:

The lipid ingredient, being fatty components, may be chosen from one or more of the following compounds:
- cocoa butter and cocoa butter alternatives, including cocoa butter equivalents (CBE), cocoa butter substitutes (CBS), cocoa butter replacers (CBR) and cocoa butter improvers (CBI),
- coconut, palmkernel oil and other similar oils characterized by being predominantly based on lauric and myristic acids,
- palm oil, shea butter, karite butter, illipe butter, mango kernel oil, sal fat and other similar fats characterized by being predominantly based on palmitic, oleic and stearic acids,
- corn oil, sunflower oil, hybrid sunflower oil, soybean oil, rapeseed oil, canola oil, olive oil, rice bran oil, cottonseed oil, arachis (peanut, groundnut) oil and other oils characterized by being predominantly based on oleic, linoleic and linolenic acids and hydrogenated to a suitable melting point,
- fish oil, tallow, lard, butterfat and other animal derived fats, and
- synthetic fats, reesterified fats, hard fats obtained by a chemical reaction of fatty acids with glycerol using no, acidic, alkaline or enzymatic catalysis,
whereby said compound(s) is/are used as a single component or mixed with each other, being either crude or refined using physical or alkaline refining, or being subjected to further processing including catalytic hydrogenation, interesterification, transesterification and fractionation.

The buffer sodium carbonate may be exchanged for carbonates, bicarbonates, acetates, gluconates, glycerophosphates, phosphates, glycinates, citrates, malates and/or tartrates of sodium, potassium or ammonium, or mixtures thereof. Most phosphates are though less suitable because their taste usually is disagreeable and difficult to mask.

The sweetener aspartame may entirely or in part be exchanged for one or more other artificial sweeteners, such as acesulfame potassium, saccharine, cyclamate, glycyrrhizine, dihydrochalcones, stevioside, thaumatin, monellin and/or neohesperidine.

The emulsifier lecithin is preferably soy lecithin and/or egg lecithin, but may be exchanged for
- a nonionic surfactant, such as poloxamer, polyoxyethylene alkyl ether, polyoxyethylene castor oil derivative, polyoxyethylene sorbitan fatty acid ester, monoglyceride, diglyceride and esther thereof, polyoxyethylene stearate, polyglycerolester of fatty acids (including polyglycerolpolyricinoleic acid (PGPR)), sorbitan fatty acid ester,
- an anionic surfactant, such as fatty acid, soap of fatty acid, lactylate, especially sodium and/or calcium stearoyllactylate, sodium lauryl sulfate and latanol,
- a zwitterionic surfactant, such as zwitterionic phospholipid, such as phosphatidylcholine and phosphatidylethanolamine,
or mixtures, fractions or derivatives thereof or with lecithin.

Optionally liquid or solid flavoring agents may be added. Examples of flavoring agents are peppermint, coffee, orange and vanilla.

### Example 3: Useful concentrations ranges

In Example 1 is disclosed a preferred embodiment and in Example 2 are disclosed embodiments with alternative excipients. Useful embodiments are obtainable within concentration ranges for the respective components of the formulation per unit dose as follows

| | |
|---|---|
| Nicotine in any form: | from 0.5 mg 10 mg as base |
| Diluent/filler and | |
| taste-masking, smoothening | |
| and flavoring agent: | from 17% to 70% (w/w) |
| Lipid ingredient: | from 20% to 50% (w/w) |
| Sweetener: | from 0.3% to 3% (w/w) |
| Buffering agent: | from 0 to 10 % (w/w) |
| Emulsifier/solubilizer: | from 0.3% to 5% (w/w) |
| Flavoring agent: | from 0% to 4% (w/w). |

The present nicotine-containing composition may be administered in combination with a second formulation for nicotine replacement therapy. This second formulation may be a device for transdermal administration of nicotine, a spray for nasal, buccal or pulmonary uptake, a chewing gum, or a dosage form for oral or peroral use or any device for administration of tobacco.

The present invention also encompasses the manufacture of a medicament for the treatment of cessation, reduction and temporary abstinence of tobacco, and for treatment of Alzheimer's disease, Parkinson's disease, ulcerative colitis and Tourette's syndrome; and weight control therapy.

## Claims

1. A nicotine-containing pharmaceutical composition, **characterized in that** a unit dose thereof comprises
| | |
|---|---|
| Nicotine in any form: | from 0.5 mg 10 mg, measured as base, |
| Diluent/filler and | |
| taste-masking, smoothening and flavoring | |
| agent: | Cocoa powder |
| | from 17% to |
| | 70% (w/w), |
| Lipid ingredient(s): | from 20% to 50% (w/w), |
| Sweetener(s): | from 0.3% to 3% (w/w), |
| Buffering agent(s): | from 0 to 10% (w/w), |
| Emulsifier(s)/solubilizer(s): | from 0.3% to 5% (w/w), |
| Flavoring agent(s): | from 0 to 4% (w/w). |

2. A nicotine-containing pharmaceutical composition according to claim 1, **characterized in that** the lipid ingredient(s) is/are chosen from
- cocoa butter and cocoa butter alternatives, including cocoa butter equivalents (CBE), cocoa butter substitutes (CBS), cocoa butter replacers (CBR) and cocoa butter improvers (CBI);
- coconut, palmkernel oil and other similar oils **characterized by** being predominantly based on lauric and myristic acids,
- palm oil, shea butter, karite butter, illipe butter, mango kernel oil, sal fat and other similar fats **characterized by** being predominantly based on palmitic, oleic and stearic acids,
- corn oil, sunflower oil, hybrid sunflower oil, soybean oil, rapeseed oil, canola oil, olive oil, rice bran oil, cottonseed oil, arachis (peanut, groundnut) oil and other oils **characterized by** being predominantly based on oleic, linoleic and linolenic acids and hydrogenated to a suitable melting point,
- fish oil, tallow, lard, butterfat and other animal derived fats, and
- synthetic fats, reesterified fats, hard fats obtained by a chemical reaction of fatty acids with glycerol using no, acidic, alkaline or enzymatic catalysis,
whereby said compound(s) is/are used as a single component or mixed with each other, being either crude or refined using physical or alkaline refining, or being subjected to further processing including catalytic hydrogenation, interesterification, transesterification and fractionation.

3. A nicotine-containing pharmaceutical composition according to claim 2, **characterized in that** the one or more lipid ingredients is/are chosen from cocoa butter equivalents (CBE), cocoa butter substitutes (CBS) and cocoa butter replacers (CBR).

4. A nicotine-containing pharmaceutical composition according to any preceding claim, **characterized in that** the one or more buffering agents is/are chosen from carbonates, bicarbonates, acetates, gluconates, glycerophosphates, phosphates, glycinates, citrates, malates and/or tartrates of sodium, potassium or ammonium, or mixtures thereof.

5. A nicotine-containing pharmaceutical composition according to any preceding claim, **characterized in that** the one or more emulsifiers/solubilisers is/are chosen from
- soy lecithin and egg lecithin,
- a nonionic surfactant, such as poloxamer, polyoxyethylene alkyl ether, polyoxyethylene castor oil derivative, polyoxyethylene sorbitan fatty acid ester, monoglyceride, diglyceride and esther thereof, polyoxyethylene stearate, polyglycerolester of fatty acids (including polyglycerolpolyricinoleic acid (PGPR)), sorbitan fatty acid ester,
- an anionic surfactant, such as fatty acid, soap of fatty acid, lactylate, especially sodium and/or calcium stearoyllactylate, sodium lauryl sulfate and latanol,
- a zwitterionic surfactant, such as zwitterionic phospholipid, such as phosphatidylcholine and phosphatidylethanolamine,
or mixtures, fractions or derivatives thereof or with lecithin.

6. A nicotine-containing pharmaceutical composition according to claim 5, **characterized in that** the one or more emulsifiers/solubilisers is/are chosen from soy lecithin and egg lecithin.

7. A nicotine-containing pharmaceutical composition according to any preceding claim, **characterized in that** the one or more sweeteners is aspartame, acesulfame potassium, saccharine, cyclamate, glycyrrhizine, dihydrochalcones, stevisoide, thaumatin, monellin and/or neohesperidine.

8. A nicotine-containing pharmaceutical composition according to any preceding claim, **characterized in that** a unit dose thereof comprises 1 - 6 mg nicotine, in any form, measured as base, 50% (w/w) cocoa powder, 44% (w/w) lipid ingredient(s), 15 mg sodium carbonate, 0,6% (w/w) aspartame and/or acesulfame potassium, and 1% (w/w) lecithin.

9. A nicotine-containing pharmaceutical composition according to anyone of the preceding claims which is formulated as an oral dosage form and which provides for delivery of nicotine through the buccal mucosa and/or other mucosa of the oral cavity.

10. Use of a nicotine-containing pharmaceutical composition according to anyone of the preceding claims for the manufacture of a medicament for nicotine replacement therapy (NRT), cessation, reduction and temporary abstinence of tobacco, and for treatment of Alzheimer's disease, Parkinson's disease, ulcerative colitis and/or Tourette's syndrome; and/or for weight control therapy.

## Patentansprüche

1. Nikotinhaltige pharmazeutische Zusammensetzung, **dadurch gekennzeichnet, dass** eine Dosierungseinheit davon Folgendes umfasst:
| | |
|---|---|
| Nikotin in beliebiger Form: | von 0,5 mg bis 10 mg, gemessen als Basis, |
| Verdünnungsmittel/Füllstoffund ein Ge- | |
| schmack maskierendes, milderndes | |
| und aromatisierendes Mittel: | Kakaopulver |
| | von 17 % bis 70 % (w/w) |
| Lipidbestandteil(e): | von 20 % bis 50 % (w/w) |
| Süßstoff(e): | von 0,3 % bis 3 % (w/w) |
| Puffermittel: | von 0 bis 10 % (w/w) |
| Emulgator(en)/Solubilisierungsmittel: | von 0,3 % bis 5 % (w/w) |
| Aromastoff(e) | von 0 bis 4 % (w/w) |

2. Nikotinhaltige pharmazeutische Zusammensetzung gemäß Anspruch 1, **dadurch gekennzeichnet, dass** der/die Lipidbestandteil(e) gewählt ist/sind aus:
- Kakaobutter und Kakaobutteralternativen, einschließlich Kakaobutter-Äquivalenten (CBE), Kakaobuttersubstituten (CBS), Kakaobutterersatzstoffen (CBR) und Kakaobutterverbesserern (CBI);
- Kokosnuss, Palmkernöl und anderen ähnlichen Ölen, **dadurch gekennzeichnet, dass** sie vorwiegend auf Laurin- und Myristinsäure basieren;
- Palmöl, Sheabutter, Karitebutter, Illipebutter, Mangokemöl, Sal (Shorea robusta)-Fett und anderen ähnlichen Fetten, **dadurch gekennzeichnet, dass** sie vorwiegend auf Palmitin-, Olein- und Stearinsäure basieren;
- Maisöl, Sonnenblumenöl, Hybrid-Sonnenblumenöl, Sojabohnenöl, Rapssamenöl, Kanolaöl, Olivenöl, Reisschalenöl, Baumwollsamenöl, Arachis (Erdnuss-, Groundnut-)Öl und anderen Ölen, **dadurch gekennzeichnet, dass** sie auf Olein-, Linol- und Linolensäure basieren und zu einem geeigneten Schmelzpunkt hydriert wurden;
- Fischöl, Talg, Schmalz, Butterfett und anderen tierisch abgeleiteten Fetten; und synthetischen Fetten, umgeesterten Fetten, Hartfetten, die durch eine chemische Reaktion von Fettsäuren mit Glycerol unter Anwendung keiner, einer sauren, alkalischen oder enzymatischen Katalyse erhalten wurden;
wobei die Verbindung(en) als Einzelkomponente oder vermischt miteinander verwendet wird/werden, die entweder roh oder mit Hilfe physikalischer oder alkalischer Veredlung verfeinert sind oder die einer weiteren Verarbeitung einschließlich einer katalytischen Hydrierung, Inter-Veresterung, Umesterung und Fraktionierung unterworfen worden sind.

3. Nikotinhaltige pharmazeutische Zusammensetzung gemäß Anspruch 2, **dadurch gekennzeichnet, dass** der eine oder mehrere Lipidbestandteile aus Kakaobutter-Äquivalenten (CBE), Kakaobuttersubstituten (CBS) und Kakaobutterersatzstoffen (CBR) gewählt wird/werden;

4. Nikotinhaltige pharmazeutische Zusammensetzung gemäß gemäß einem der vorhergehenden Ansprüche, **dadurch gekennzeichnet, dass** das eine oder mehrere Puffermittel aus Carbonaten, Bicarbonaten, Acetaten, Gluconaten, Glycerophosphaten, Phosphaten, Glycinaten, Citraten, Malaten und/oder Tartraten von Natrium, Kalium oder Ammonium, oder Mischungen davon gewählt wird/werden.

5. Nikotinhaltige pharmazeutische Zusammensetzung gemäß einem der vorhergehenden Ansprüche, **dadurch gekennzeichnet, dass** der/die eine oder mehrere Emulgatoren/- Solubilisierungsmittel gewählt wird/werden aus:
- Sojalecithin und Eierlecithin;
- einem nichtionischen Tensid, wie Poloxamer, Polyoxyethylenalkylether, Polyoxyethylen-Castorölderivat, Polyoxyethylen-Sorbitanfettsäureester, Monoglycerid, Diglycerid und Ester davon, Polyoxyethylenstearat, Polyglycerolester von Fettsäuren (einschließlich Polyglycerolpolyricinoleinsäure (PGPR)), Sorbitanfettsäureester;
- einem anionischen Tensid, wie Fettsäure, Seife von Fettsäure, Lactylat, insbesondere Natrium- und/oder Calciumstearoyllactylat, Natriumlaurylsulfat und Latanol;
- einem zwitterionischen Tensid, wie zwitterionischem Phospholipid, wie Posphatidylcholin und Phosphatidylethanolamin;
oder Mischungen, Fraktionen oder Derivaten davon oder mit Lecithin.

6. Nikotinhaltige pharmazeutische Zusammensetzung gemäß Anspruch 5, **dadurch gekennzeichnet, dass** der eine oder mehrere Emulgator/Solubilisierungsmittel gewählt wird/- werden aus Sojalecithin und Eierlecithin.

7. Nikotinhaltige pharmazeutische Zusammensetzung gemäß einem der vorhergehenden Ansprüche, **dadurch gekennzeichnet, dass** der eine oder mehrere Süßstoffe Aspartam, Acesulfamkalium, Saccharin, Cyclamat, Glycyrrhizin, Dihydrochalkone, Stevisoid, Thaumatin, Monellin und/oder Neohesperidin ist.

8. Nikotinhaltige pharmazeutische Zusammensetzung gemäß einem der vorhergehenden Ansprüche, **dadurch gekennzeichnet, dass** eine Einheitsdosis davon 1 - 6 mg Nikotin in beliebiger Form, gemessen als Basis, 50 % (w/w) Kakaopulver, 44 % (w/w) Lipidbestandteil(e), 15 mg Natriumcarbonat, 0,6 % (w/w) Aspartam und/oder Acesulfamkalium und 1 % (w/w) Lecithin umfasst.

9. Nikotinhaltige pharmazeutische Zusammensetzung gemäß einem der vorhergehenden Ansprüche, welche als eine orale Dosierform formuliert ist und welche die Abgabe von Nikotin durch die Mundschleimhaut und/oder eine andere Schleimhaut der Mundhöhle vorsieht.

10. Verwendung einer nikotinhaltigen pharmazeutischen Zusammensetzung gemäß einem der vorhergehenden Ansprüche für die Herstellung eines Medikaments für die Nikotin-Ersatztherapie (NRT), Einstellung, Reduzierung und zeitweilige Abstinenz von Tabak, und für die Behandlung von Alzheimer-Krankheit, Parkinson-Krankheit, ulzerativer Kolitis und/oder Tourette-Syndrom; und/oder für die Therapie zur Kontrolle des Gewichts.

## Revendications

1. Composition pharmaceutique contenant de la nicotine, **caractérisée en ce qu'**une dose unitaire comprend
| | |
|---|---|
| de la nicotine sous une forme quelconque: | de 0,5 mg à 10 mg, mesurée en tant |
| | que base |
| un diluant/charge, et un agent masquant le goût, | |
| lissant et aromatisant: | poudre de cacao, de 17 % à 70 % en |
| | masse |
| un ou plusieurs ingrédients lipidiques: | de 20 % à 50 % en masse, |
| un ou plusieurs édulcorants: | de 0,3 % à 3 % en masse, |
| un ou plusieurs agents de tamponnage: | de 0 à 10 % en masse; |
| un ou plusieurs émulsionnants/solubilisants: | de 0,3 % à 5 % en masse, |
| un ou plusieurs agents aromatisants: | de 0 à 4 % en masse. |

2. Composition pharmaceutique contenant de la nicotine selon la revendication 1, **caractérisée en ce que** le ou les ingrédients lipidiques est/sont choisis parmi:
- du beurre de cacao et des variantes du beurre de cacao, comprenant des équivalents du beurre de cacao (EBC), des substituts du beurre de cacao (SBC), des produits de remplacement du beurre de cacao (RBC) et des produits améliorant le beurre de cacao (ABC);
- l'huile de noix de coco, de palmiste et d'autres huiles similaires **caractérisées par le fait qu'**elles sont de façon prédominante à base d'acides laurique et myristique,
- l'huile de palme, l'huile de karité, le beurre de karité, le beurre d'illipé, l'huile de noix de mangue, la graisse de sal et d'autres graisses similaires **caractérisées par le fait qu'**elles sont de façon prédominante à base d'acides palmitique, oléique et stéarique,
- l'huile de maïs, l'huile de tournesol, l'huile de tournesol hybride, l'huile de soja, l'huile de colza, l'huile de canola, l'huile d'olive, l'huile de son de riz, l'huile de graines de coton, l'huile d'arachide (cacahuète), et d'autres huiles **caractérisées en ce qu'**elles sont de façon prédominante à base d'acides oléique, linoléique et linolénique, et hydrogénées jusqu'à un point de fusion approprié,
- l'huile de poisson, le suif, le lard, la matière grasse du beurre, et d'autres matières grasses d'origine animale, et
- des matières grasses synthétiques, des matières grasses réestérifiées, des graisses dures obtenues par réaction chimique d'acides gras avec du glycérol n'utilisant pas de catalyse acide, alcaline ou enzymatique,
le ou lesdits composés étant utilisés sous forme de constituants isolés ou mélangés entre eux, bruts ou raffinés par raffinage physique ou alcaline ou soumis à un traitement supplémentaire comprenant une hydrogénation catalytique, une interestérification, une transestérification et un fractionnement.

3. Composition pharmaceutique contenant de la nicotine selon la revendication 2, **caractérisée en ce que** le ou les ingrédients lipidiques est/sont choisis parmi des équivalents du beurre de cacao (ECB), des substituts du beurre de cacao (SBC) et des produits de remplacement du beurre de cacao (RBC).

4. Composition pharmaceutique contenant de la nicotine selon l'une quelconque des revendications précédentes, **caractérisée en ce que** le ou les agents de tamponnage est/sont choisis parmi des carbonates, des bicarbonates, des acétates, des gluconates, des glycérophosphates, des phosphates, des glycinates, des citrates, des malates et/ou des tartrates de sodium, de potassium ou d'ammonium, ou leurs mélanges.

5. Composition pharmaceutique contenant de la nicotine selon l'une quelconque des revendications précédentes, **caractérisée en ce que** le ou les émulsionnants/solubilisants est/sont choisis parmi:
- la lécithine de soja et la lécithine d'oeufs,
- un agent tensioactif non ionique, comme un poloxamère, un éther alkylique de polyoxyéthylène, un dérivé polyoxyéthylénique d'huile de ricin, un ester d'acide gras de polyoxyéthylènesorbitane, un monoglycéride, un diglycéride et leurs esters, un stéarate de polyoxyéthylène, un ester de polyglycérol d'acides gras (comprenant l'acide polyglycérolpolyricinoléique (PGPR)), un ester d'acide gras du sorbitane,
- un agent tensioactif anionique, comme un acide gras, un savon d'acide gras, un lactylate, en particulier un stéaroyllactylate de sodium et/ou de calcium, le laurylsulfate de sodium et le latanol,
- un agent tensioactif zwitterionique, comme un phospholipide zwitterionique, comme la phosphatidylcholine et la phosphatidyléthanolamine,
ou leurs mélanges, fractions ou dérivés ou avec la lécithine.

6. Composition pharmaceutique contenant de la nicotine selon la revendication 5, **caractérisée en ce que** le ou les émulsionnants/solubilisants est/sont choisis parmi la lécithine de soja et la lécithine d'oeufs.

7. Composition pharmaceutique contenant de la nicotine selon l'une quelconque des revendications précédentes, **caractérisée en ce que** le ou les édulcorants sont l'aspartame, l'acésulfame potassique, la saccharine, le cyclamate, la glycyrrhizine, des dihydrochalcones, le stévioside, la thaumatine, la monelline et/ou la néohespéridine.

8. Composition pharmaceutique contenant de la nicotine selon l'une quelconque des revendications précédentes, **caractérisée en ce qu'**une dose unitaire comprend 1-6 mg de nicotine, sous une forme quelconque, mesurée en tant que base, 50 % en masse de poudre de cacao, 44 % en masse d'un ou plusieurs ingrédients lipidiques, 15 mg de carbonate de sodium, 0,6 % en masse d'aspartame et/ou d'acésulfame potassique, et 1 % en masse de lécithine.

9. Composition pharmaceutique contenant de la nicotine selon l'une quelconque des revendications précédentes, qui est formulée en une forme galénique orale qui assure la délivrance de nicotine par l'intermédiaire de la muqueuse buccale et/ou d'une autre muqueuse de la cavité orale.

10. Utilisation d'une composition pharmaceutique contenant de la nicotine selon l'une quelconque des revendications précédentes pour la fabrication d'un médicament destiné à une thérapie de remplacement de la nicotine (TRN), d'arrêt, de réduction et d'abstinence temporaire du tabac, et au traitement de la maladie d'Alzheimer, de la maladie de Parkinson, de la colite ulcéreuse et/ou du syndrome de Tourette; et/ou à une thérapie de maîtrise de poids.
